# EUROPEAN PATENT APPLICATION

(11) **EP 4 137 648 A1**
(43) Date of publication of application: **22.02.2023**
(21) Application number: 22189771.3
(22) Date of filing: 10.08.2022
(51) Int. Cl.: E03D 9/03, E03B 1/04, E03C 1/18, E03D 1/32, E03D 5/02

(54) **OZONE GAS MITIGATION DEVICES FOR PLUMBING FIXTURES**

(30) Priority: 19.08.2021 US 202163234893 P; 28.10.2021 US 202163272987 P; 08.08.2022 US 202217883169
(71) Applicant: Kohler Co., Kohler, WI 53044 (US)
(72) Inventor: KURU, William, Plymouth, 53073 (US); GARRELS, Clayton, Sheboygan, 53081 (US); DENZIN, Peter, Glenbeulah, 53023 (US)
(74) Representative: Barker Brettell LLP

(57) **Abstract**

An ozone mitigation system reduces the amount of ozone that is expelled from a toilet or another water consuming appliance. The ozone mitigation system may include an ozone generator in communication with a tank of the water consuming appliance. An air source and a power source may be connected to the ozone generator. An ozone flow restrictor or ozone eliminator is operable inside of the tank and configured to modify a flow of ozone from the ozone generator through the toilet tank.

## Description

This application claims priority benefit of Application No. 17/883,169 filed August 8, 2022, Provisional Application No. 63/272,987 filed October 28, 2021, and Provisional Application No. 63/234,893 filed August 19, 2021, which are hereby incorporated by reference in their entirety.

### FIELD

The present application relates generally to the reduction or mitigation of ozone in toilets or other appliances including plumbing fixtures.

### BACKGROUND

Ozone, or trioxide or O₃, is an inorganic molecule and reactive gas. It may be pale blue in color and present a distinctive odor. It is an allotrope of oxygen and less stable than oxygen. Ozone may be formed naturally in the atmosphere by reaction with ultraviolet light from the sun and electrical discharges in the atmosphere.

Ozonated water is a powerful disinfectant that can be generated on-site. On-site generation has advantages. One advantage is that there are no consumable chemicals required. One problem is that ozone has a slow and limited solubility in water. As such, ozone gas likely escapes from the water surface and into the environment.

Some people consider the odor of ozone to be unpleasant. In addition, some governments have enacted regulations for ozone in air and may set limits on ozone emissions and/or ozone exposure.

The following embodiments provide ozone in controlled environments to harness the beneficial properties of the gas, while shielding the release of ozone near humans and animals to avoid or mitigate any potential harmful effects. The following embodiments may improve the dissolution of ozone and manage the escape of ozone gas from the water.

### BRIEF DESCRIPTION OF THE DRAWINGS

Exemplary embodiments are described herein with reference to the following drawings, according to an exemplary embodiment.
FIG. 1 illustrates an example toilet for use with an ozone mitigation system.
FIG. 2 illustrates an example passive ozone mitigation system.
FIG. 3A illustrates an example ozone mitigation system including an obstacle pattern.
FIG. 3B illustrates an embodiment the obstacle pattern of FIG. 3A.
FIG. 3C illustrates another embodiment the obstacle pattern of FIG. 3A.
FIG. 4 illustrates an example ozone mitigation system including a baffle pattern.
FIG. 5 illustrates an example ozone mitigation system including one or more paddles.
FIG. 6 illustrates an example active ozone mitigation system.
FIG. 7 illustrates an example ozone mitigation system including an impellor.
FIG. 8 illustrates an example ozone mitigation system including a pump.
FIG. 9 illustrates an example ozone mitigation system incorporated with a flush valve.
FIG. 10 illustrates an example ozone mitigation system including an elimination device.
FIG. 11 illustrates an example ozone mitigation system including a light source.
FIG. 12 illustrates an example ozone mitigation system including a filter.
FIG. 13 illustrates an example ozone mitigation system including a ventilation device.
FIG. 14 illustrates an example ozone mitigation system including an external venting system.
FIG. 15 illustrates an example ozone mitigation system including a sump venting system.
FIG. 16 illustrates an example in-wall tank including an ozone mitigation system.
FIG. 17 illustrates an example seal for a flush control for an in-wall tank.
FIG. 18 illustrates an example controller for any of the ozone mitigation systems.
FIG. 19 illustrates an example flow chart for the controller of FIG. 18.
FIG. 20 illustrates another example flow chart for the controller of FIG. 18.
FIG. 21 illustrates another example flow chart for the controller of FIG. 18.
FIG. 22 illustrates another ozone mitigation device.
FIG. 23 illustrates another ozone mitigation device including an ozone dissipation path.
FIG. 24 illustrates another ozone mitigation device with a bypass path.
FIG. 25 illustrates a sink with ana example ozone mitigation path.
FIG. 26A-C illustrate example appliances for use with an ozone mitigation system.
FIG. 27 illustrates another example flow chart for an ozone mitigation device.

### DETAILED DESCRIPTION

The following embodiments include ozonated water that is generated at or near a toilet and delivered to various areas of the toilet bowl for disinfection. The following embodiments may improve the dissolution of ozone for a toilet, specifically, and manage the escape of ozone gas from the water.

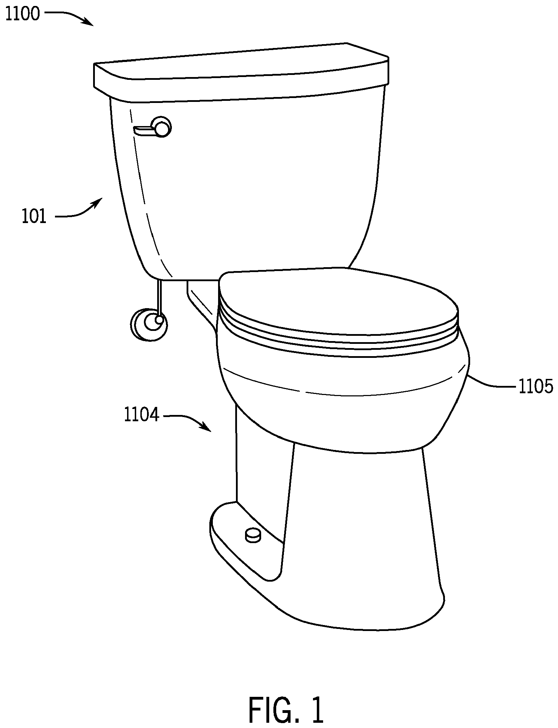

FIG. 1 illustrates an example toilet for use with an ozone mitigation system. Referring to FIG. 1, a toilet 1100 with the ozone mitigation system is illustrated according to an exemplary embodiment of the present disclosure. The toilet 1100 may include a tank (e.g., container, reservoir, etc.), shown as a tank 101, and a pedestal (e.g., base, stand, support, etc.), shown as a pedestal 1104. The tank 101 may be coupled to, and supported by, the pedestal 1104, which may be positioned on a floor. In some embodiments, the tank 101 and the pedestal 1104 may be formed together as a single component. In some other examples, the tank 101 may be mounted nearby on a wall, or within a wall. The tank 101 is configured to receive water (e.g., via a fill valve of the toilet 1100, etc.) and store the water in between flushes. The pedestal 1104 includes a bowl 1105 and may be configured to receive the water from the tank 101 to flush contents of bowl into a sewage line. In some embodiments, the pedestal 1104 may be mounted on the wall of a lavatory and the bowl may be configured to receive water from a fluid supply source such as a household water supply.

The bowl 1105 of the pedestal 1104 includes a sump (e.g. a receptacle) and an outlet opening, wherein water and waste is collected in the sump until being removed through the outlet opening, such as when the contents of the bowl are flushed into a sewage line. The toilet 1100 further includes a trapway, and the trapway may be fluidly connected to the bowl via the sump. The trapway fluidly connects the sump to the outlet opening.

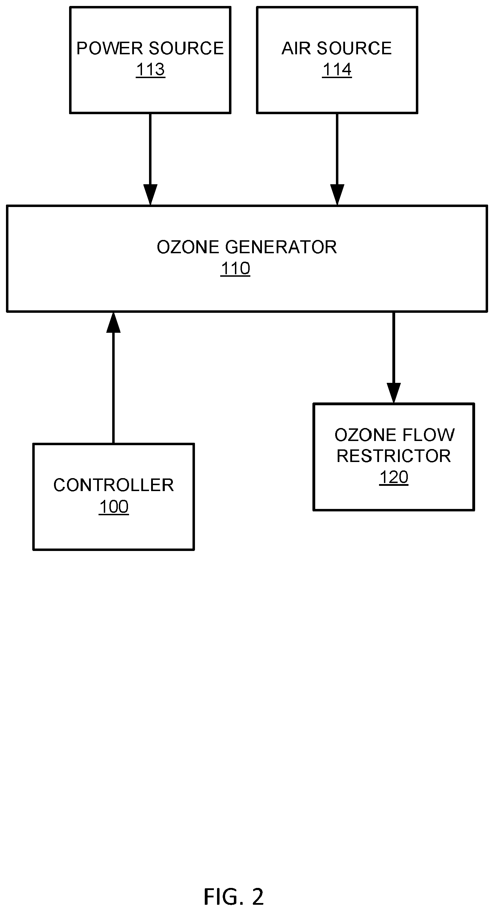

FIG. 2 illustrates an example passive ozone mitigation system for the tank 101. The ozone mitigation system may include an ozone generator 110 including inputs comprising a power source 113 and an air source 114. The ozone generator 110 is in communication with the tank 101 (e.g., submerged in the tank below a fill water level 125). The ozone generator 110 may receive commands from a controller 100. The ozone mitigation system may include an ozone flow restrictor 120 operable inside of the toilet tank 101 and configured to modify a flow of ozone from the ozone generator 110 through the toilet tank 101 as the ozone is dissolved in the water of the toilet tank 101. The ozone mitigation system is for mitigating the release of ozone in the ambient environment of the toilet 1100. Additional, different, or fewer components may be included.

The ozone formed by the ozone generator 110 may include micro-bubbles that are suspended in the water of the tank 101. The micro-bubbles may have a diameter of 50 microns or smaller.

Ozone may be formed by the ozone generator 110 using a variety of techniques, including corona discharge, ultraviolet light, cold plasma, and other techniques. For example, a corona charger may be configured to accumulate electric charge from the power source 113 and apply the electric charge to air from the air source 114.

In corona discharge, a corona discharge tube or an ozone plate is used. For example, a high voltage may be applied to an electrode in discharge tube or on the ozone plate. A corona discharge is an electrical discharge caused by the ionization of air surrounding the conductor carrying the high voltage. The air around the conductor undergoes an electrical breakdown to become conductive (e.g., temporarily) so that charge can leak off of the conductor and into the air. A corona occurs at locations where the strength of the electric field (potential gradient) around a conductor exceeds the dielectric strength of the air.

The ozone generator 110 may use air source 114, which may include only ambient air. The air may be provided under stored pressure or a differential pressure in the ozone generator 110. Ambient air may correspond to ozone production in a predetermined concentration range (e.g., 3-6%). Alternatively, an oxygen concentrator may be used to increase the concentration of oxygen in the air source. Pure oxygen may be used.

The water vapor content of the air source 114 may be regulated by an air dryer. Less water vapor in the air supply may reduce the production of byproducts such as nitrogen oxides. The air dryer may eliminate nitric acid formation by removing water vapor and may also increase ozone production. Nitric acid may be harmful in certain situations.

The corona discharge may occur in a sealed container of the ozone generator 110. The sealed container may include a seal to prevent entry of water from the tank 101. The sealed container may include one or more valves to allow air into the cavity of the sealed container and ozone out of the sealed container.

In another technique, ozone may be produced by ultraviolet light. Such an ozone generator 110 includes a light source that generates a narrow-band ultraviolet light. The narrow-band ultraviolet light may be less than the spectrum of light produced by the sun. Ultraviolet light may produce ozone at a lower concentration (e.g., 1%) than corona techniques. Ultraviolet light ozone generates may exclude both air dryers and oxygen concentrators.

In another technique, ozone may be produced by cold plasma. Such an ozone generator 110 includes a dielectric barrier discharge configured to generate plasma. Pure oxygen gas is supplied to the plasma and the oxygen molecules are split into single atoms, which recombine into groups of three, forming ozone, or O₃. Cold plasma techniques may produce high concentrations of ozone (e.g., 5% or greater) using a small amount of space.

In another technique, an electrolytic ozone generate produces ozone by splitting water molecules. Such an ozone generator 110 includes a water electrolysis device that splits water molecules into H₂, O₂, and O₃. The hydrogen gas, H₂, may be removed to leave oxygen and ozone as the only products of the reaction. Electrolytic ozone generation may produce at higher concentrations (20-30%) than the corona discharge technique. The electrolytic techniques may also avoid nitrogen gases.

The controller 100 may send commands to the ozone generator 110. For example, the commands may initiate the generation of ozone. The commands may be triggered by a time schedule (e.g., once every predetermined time period or at certain times of day). The commands may be triggered by a flush cycle. That is, the controller 100 may send a command to the ozone generator 110 to generate ozone when the tank is filled.

The controller 100 may send a command to the ozone generator 110 to generate ozone in response to a user input. For example, a flush cycle may be initiated by operation of an actuator. The actuator may be a button configured to initiate the flush cycle when depressed (or pulled) a predetermined distance or when touched, a lever configured to activate when rotated a predetermined angular travel, or any suitable device configured to activate based on an input manipulation by a user. In some embodiments, the actuator may be a sensor (e.g., a proximity sensor) and the flush cycle may be automatically initiated (e.g., by a controller) based on sensor data received from the sensor.

The controller 100 may also receive sensor data as feedback for one or more conditions in proximity to the tank 101 or the toilet. For example, the sensor data may indicate the presence of ozone. The sensor may be an ozone sensor. The ozone sensor may receive air samples and provide one or more tests on the air samples. The ozone sensor may determine a proportion of light that is absorbed by air in the tank 101 or in the proximity of the toilet 1100. The proportion of light may be indicative of a level of ozone present. The proportion off light may indicate a concentration of ozone. A scrubber within ozone sensor may reset the sensor to test a subsequent air sample.

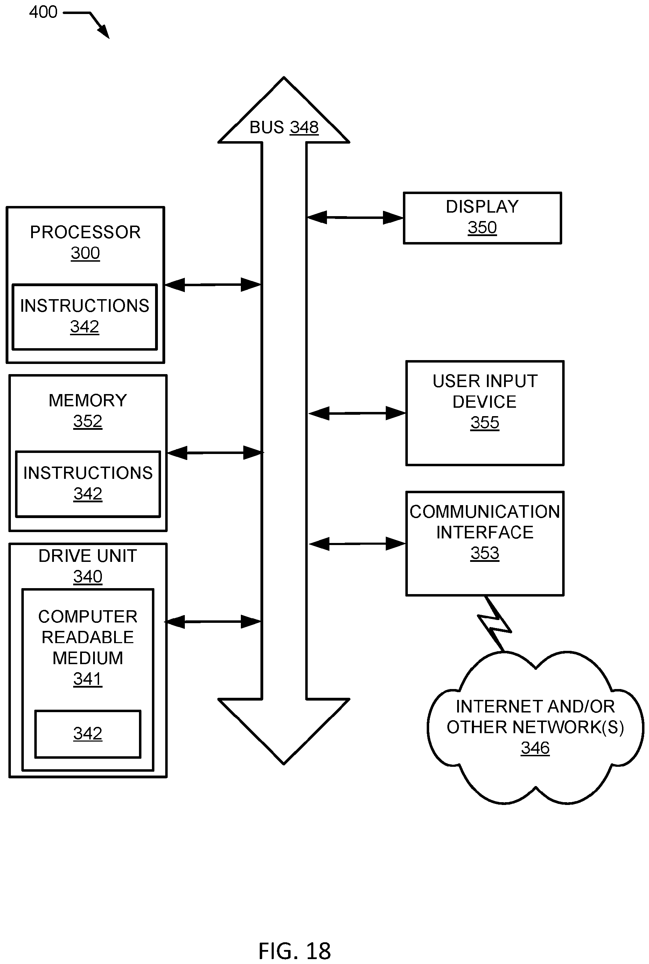

In the instance of automatic initiation of the flush cycle, the controller 100 (e.g., as described herein with respect to FIG. 18), may receive sensor data indicative of usage of the toilet. For example, the controller 100 may be in communication with a sensor configured to detect the presence of a user and initiate the flush cycle in response to a user leaving the vicinity of the toilet.

The sensor may include any type of sensor configured to detect certain actions and/or to provide functionality (e.g., dispensing, flushing, etc.). The sensor may include any type of sensor configured to detect certain conditions and/or to provide functionality. Odor sensors, proximity sensors, and motion sensors are non-limiting examples of sensors that may be employed with the systems of this application. Odor sensors, such as volatile organic compound (VOC) sensors, may be employed to detect organic chemicals and compounds, both human made and naturally occurring chemicals/compounds. Proximity sensors may be employed to detect the presence of an object within a zone of detection without physical contact between the object and the sensor. Electric potential sensors, capacitance sensors, projected capacitance sensors, and infrared sensors (e.g., projected infrared sensors, passive infrared sensors) are non-limiting examples of proximity sensors that may be employed with the systems of this application. Motion sensors may be employed to detect motion (e.g., a change in position of an object relative to the objects surroundings). Electric potential sensors, optic sensors, radio-frequency (RF) sensors, sound sensors, magnetic sensors (e.g., magnetometers), vibration sensors, and infrared sensors (e.g., projected infrared sensors, passive infrared sensors) are non-limiting examples of motion sensors that may be employed with the systems of this application.

In another example, the sensor may include a sensor configured to detect a water level. The sensor may include a float sensor, a pressure level sensor, an ultrasonic water level transmitter, a capacitance level sensor (e.g., an RF sensor), and a radar level sensor. Further, an optical sensor may be used to determine a water level.

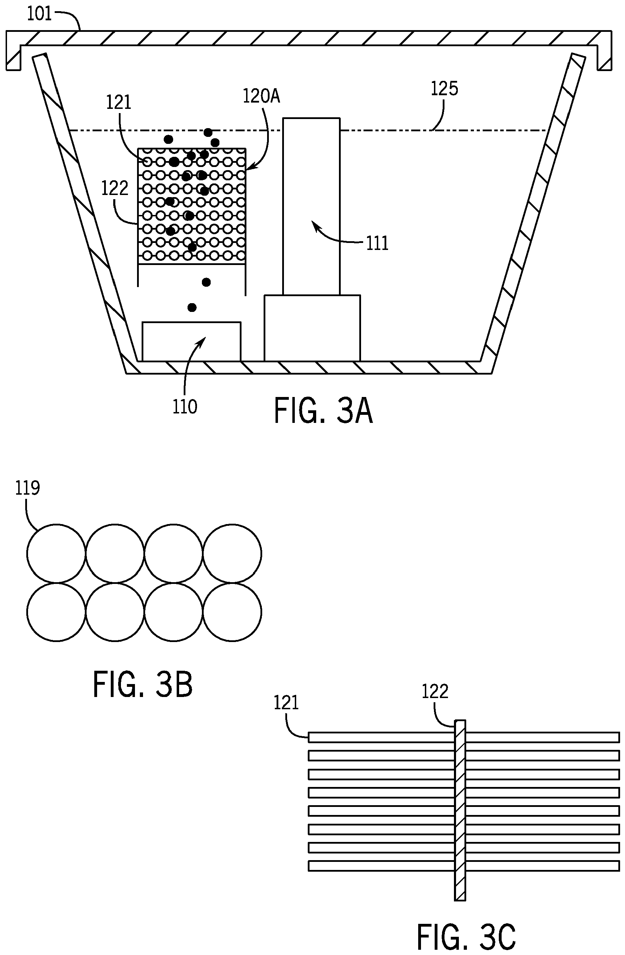

FIG. 3A illustrates an example ozone mitigation system including an obstacle pattern to slow the flow of ozone through the toilet tank 101 to improve dissolution of the ozone in the water. In one embodiment the passive ozone mitigation system includes an ozone path obstacle system 120A. For example, ozone path obstacle system 120A is provided in a toilet tank 101 in proximity to ozone generator 110 and a flush valve 111. Additional, different, or fewer components may be included.

The ozone path obstacle system 120A may include one or more physical obstacles to the ozone generated by the ozone generator 110 and rising through the water in the tank 101. The obstacles may include pegs 121 (e.g., shafts) arranged in a grid that forms rows and/or columns. The rows and/or columns may be staggered so that a center of one peg 121 is not exactly aligned vertically or horizontally with adjacent pegs. This arrangement creates a natural serpentine shape so that the ozone move back and forth as it traverses the natural serpentine shape around the pegs 121.

The pegs 121 may be cylindrical in shape and connected via a plate 122. In FIG. 3A, multiple plates 122 may be used with at least one plate parallel to the longitudinal direction of the pegs 121 and at least one plate perpendicular to the longitudinal direction of the pegs 121.

As an alternative or addition to the pegs 121, a column or ring of beads may be used to slow the flow of ozone. A bead may be spherical. The beads may be arranged in a grid that forms rows and/or columns. The grid may be three-dimensional such that the beads are arranged in a first direction, a second direction, and a third direction. The beads may be arranged in various other positions such as a ring.

The beads and/or the pegs 121 may be treated with a chemical or other substance configured to break down ozone. In one example, the beads and/or the pegs 121 may be treated with a photocatalyst that assists in breaking down ozone in the presence of ultraviolet light. One example photocatalyst may be titanium oxide (TiO₂).

In one example, a group of beads 119 are placed in a chamber as shown in FIG. 3B. A variety of packing patterns may be used. As illustrated in FIG. 3B, a loose packing may be used which maximizes the space between beads 119. Alternatively, a tight packing may be used which minimizes the space between beads 119. The ozone may travel through the spaces within the beads 119. However, the distance traveled for the ozone is increase, which slows down the ozone and increases the dissolution of the ozone.

Returning, the longitudinal rods or pegs, FIG. 3C illustrates an arrangement for the pegs 121 and plate 122 in an example where the plate 122 is secured at substantially a center of the pegs 121. The pegs 121 may be cylindrical or circular in cross section. Other shapes such as rectangles, squares, hexagons or other shapes may be used. In one example, a half moon is used as the cross section with the concave portion pointed down in the direction of gravity in order to create a pocket that captures and slows the rising ozone.

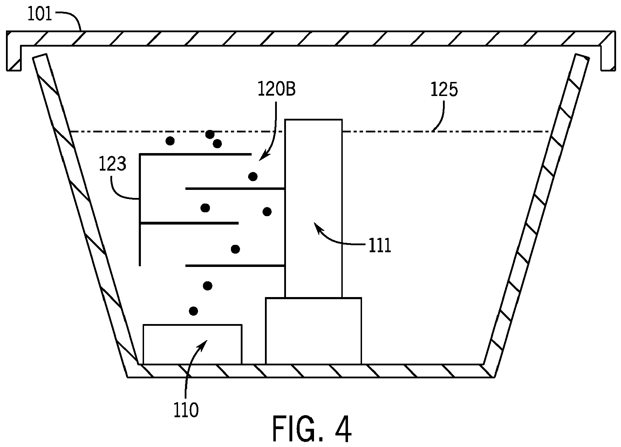

FIG. 4 illustrates an example ozone mitigation system including a baffle system 123. The baffles include plates that cover the area above the ozone generator 110. The baffle system 123 includes multiple layers. A first layer of the baffle system 123, for example, the lowest baffle may be open (e.g., include a gap or space) for ozone to pass on a first side, for example the left side (farther from the flush valve 111). The second layer (subsequent layer) of the baffle system 123 may be open (e.g., include a gap or space) for ozone to pass on a second side, for example the right side (closer to the flush valve 111). A similar pattern may be used for other baffles (e.g., third layer, fourth layer, and so on) until a gap 120B in the upper layer.

The ozone rises vertically from the ozone generator 110 enters a chamber between the first and second baffles and is slowed as the bubbles travel horizontally through the space created by the baffles. Eventually, ozone reaches the opening to the next chamber between the second and third baffles and is slowed as it travels horizontally in the opposite direction. The baffles may be placed at an angle with the horizontal, such as 1, 5, 10, or 20 degrees. The angle may be selected based on a desired speed of the ozone traveling through the water of the tank, or similarly, based on a level of desired ozone restriction. As the baffles are steeper (greater angle with the horizontal), the ozone travels faster.

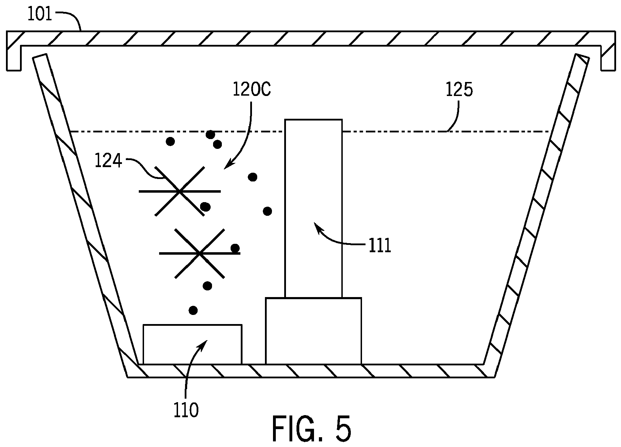

FIG. 5 illustrates an example ozone mitigation system 120C including one or more paddles 124. The paddles 124 may include arms configured to rotate about an axis. The arms may include cups or concave portions configured to capture the ozone rising from the ozone generator 110. As the ozone bubbles enter the partial compartment formed by the paddles 124, the ozone bubbles cause the paddles 124 to rotate. The ozone places a torque on the paddles 124, which slowly rotate to release the ozone. In another example, the paddles 124 may be driven by a shaft, such as a shaft coupled to a motor or other drive mechanism. The drive mechanism may slowly rotate the shaft and the paddles 124 to slowly release the ozone. The controller 100 may generate an instruction for the ozone generator 110 and/or the paddles 124. The instruction may be in response to a flush cycle of the toilet. For example, when water is replenished to the tank, the controller 100 causes the ozone generator 110 to generate ozone and the paddles 124 to rotate to slow the path of the generated ozone though the water in the tank.

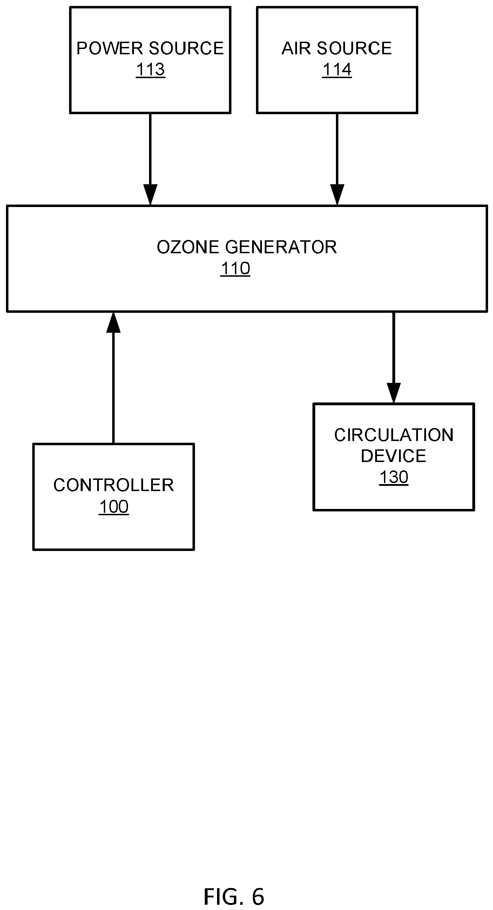

FIG. 6 illustrates an example active ozone mitigation system for the tank 101. As described in other embodiments, the ozone mitigation system may include an ozone generator 110 including inputs comprising a power source 113 and an air source 114. The ozone generator 110 is in communication with the tank 101 (e.g., submerged in the tank below the water level 125). The ozone generator 110 may receive commands from a controller 100. The ozone mitigation system may include circulation device 130 operable inside of the toilet tank 101 and configured to modify a flow of ozone from the ozone generator 110 through the toilet tank 101 as the ozone is dissolved in the water of the toilet tank 101. The circulation device 130 is for mitigating the release of ozone in the ambient environment of the toilet 1100. Additional, different, or fewer components may be included.

The circulation device 130 may include a powered device that circulates water within the tank 101. The powered device may be a motor. Examples for the circulation device 130 may include an impellor, an agitator, or a pump. The circulation device 130 may include a compartment that traps the ozone and slowly rotates the ozone until eventually releasing the ozone. The circulation device 130 may push the water including the ozone into a path that delays the release of the ozone. The circulation device 130 may open and close a chamber to capture and release the ozone.

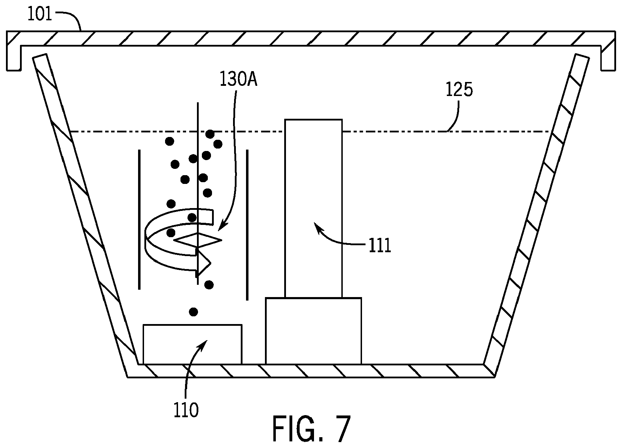

FIG. 7 illustrates an example ozone mitigation system including an impellor 130a. The impellor 130a may include a rotor or other rotating part that includes one or more fins or blades. The rotor is configured to accelerate or otherwise propel fluid (e.g., water) from an inside portion near a center of rotation of the rotor to an outside portion, which drive the water in a direction perpendicular to the center of rotation.

As the fins rotate, the water is pushed downward. The path of the ozone to the top of the water is slowed. Some ozone bubbles may evade the impellor 130a, but on average the time that the ozone bubbles spend under water is increased by the impellor 130a. The controller 100 may generate an instruction for the ozone generator 110 and/or the impellor 130a. The instruction may be in response to a flush cycle of the toilet. For example, when water is replenished to the tank, the controller 100 causes the ozone generator 110 to generate ozone and the impellor 130a to rotate to slow the path of the generated ozone though the water in the tank.

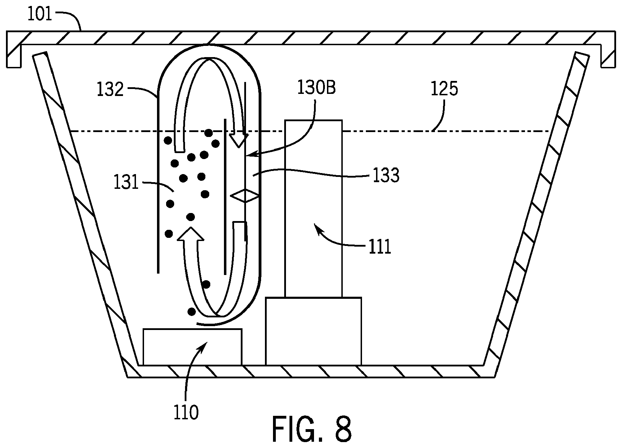

FIG. 8 illustrates an example ozone mitigation system including a pump 130b. Some example pumps include impellors as described above. Others may include submersible pumps, displacement pumps, diaphragm pumps, or piston pumps.

The pump 130b may be arranged vertically to create a vertical circular path in the tank 101. As shown in FIG. 8 the vertical circular path may include an ascending chamber 131, an ozone recirculation chamber 132, and a descending chamber 133.

As ozone is released by the ozone generator 110, a substantial portion of the ozone travels through the ascending chamber 131 into the ozone recirculation chamber 132. The ozone recirculation chamber 132 is closed (e.g., sealed) from the air space above the water in the tank. The pump 130b pushes water down through the descending chamber 133. The descending water absorbs and dissolves ozone from the ozone recirculation chamber 132, which increases the amount of ozone dissolves in the water of the tank and reduces the amount of ozone that ultimately is released from the tank.

The controller 100 may generate an instruction for the ozone generator 110 and/or the pump 130b. The instruction may be in response to a flush cycle of the toilet. For example, when water is replenished to the tank, the controller 100 causes the ozone generator 110 to generate ozone and the pump 130b to pump water against the path of the ozone to slow the path of the generated ozone though the water in the tank.

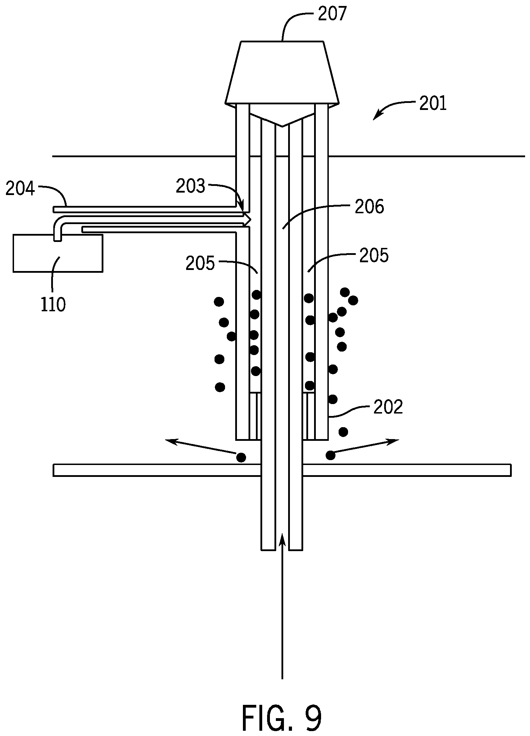

FIG. 9 illustrates an example ozone mitigation system incorporated with a fill valve assembly 201 as an example of an ozone flow restrictor or a circulation device. The fill valve assembly 201 is configured to draw ozone from the ozone generator 110 and expel the ozone to the toilet tank at a predetermined position. The predetermined position may be in proximity to the bottom of the toilet tank. The predetermined position may be in the bottom half of the toilet tank or halfway to the fill water level.

In one example, the fill valve assembly 201 includes a network of internal channels configured to increase the path of ozone through the toilet tank 101. The fill valve assembly 201 may include a water inlet channel 205, a line supply of water 206, and an ozone inlet channel 204. A seal 207 may provide an atmospheric break to prevent back siphoning of water into the water supply (line supply 206). Additional, different, or fewer components may be included.

The fill valve assembly 201 may include additional components not illustrated. Such components may include a float to turn off the fill valve at a predetermined height and/or turn on the fill valve at a predetermined height. Such components may include a refill tube connected to the fill valve assembly 201 by a fill tube. Such components may include an actuator for activating the fill valve to fill the tank. Example actuators are described herein and include manual levers and automatic actuators triggered by the controller and/or sensor readings. The refill tube may be coupled to the top of the fill valve through the top of the flush valve and into the toilet bowl.

The water inlet channel 205 is connected to the line supply of water 206. The line supply of water 206 may be connected to the plumbing system of a home or building via a shutoff valve external to the toilet.

As the ozone generator 110 generates ozone (e.g., bubbles), the ozone is provided to the ozone inlet channel 204 connected to the water inlet channel 205 via a venturi opening 203. The venturi opening 203 includes at least a portion of the water inlet channel 205 that creates a differential pressure to draw ozone through the ozone inlet channel 204. The venturi opening 203 includes a narrowed section of passage having a smaller cross section than a preceding portion of the passage. The narrowed section causes an increase in the velocity of the flow of air and/or water (e.g., ozone). Through the conservation of energy, the increase in the velocity of the flow of air causes a decrease in pressure near the venturi opening 203. The decrease in pressure or differential pressure creates a force that pulls the ozone from the ozone inlet channel 204.

The path of the ozone through the water inlet channel 205 is indirect. After leaving the ozone inlet channel 204, the ozone travels down (e.g., in the direction of gravity) through the water inlet channel 205. The increased time improves the dissolution of ozone in the water and reduces the amount of ozone that could potentially escape the water in the tank.

Optionally, to further regulate the flow of ozone through the tank. An ozone choke device 202 may be included at the outlet of the water inlet channel 205. The ozone choke device 202 may be configured to slow the flow of ozone from the fill valve assembly 201. The ozone choke device 202 may include a mesh such as a grid-like pattern of metal or plastic with small openings sized to allow small ozone bubbles to pass through. The ozone choke device 202 may include a filter.

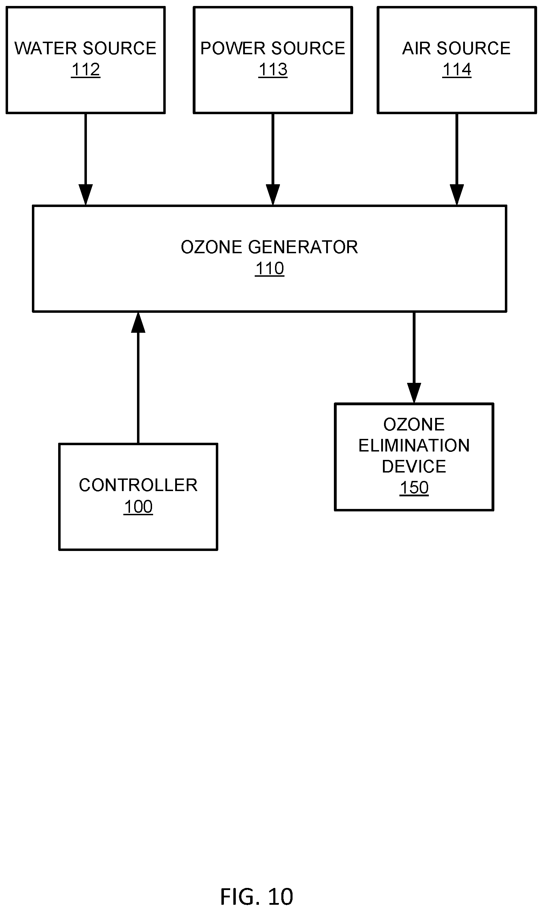

FIG. 10 illustrates an example ozone mitigation system including an ozone elimination device 150. The ozone mitigation system may include an ozone generator 110 including inputs comprising a power source 113 and an air source 114. The ozone generator 110 is in communication with the tank 101 (e.g., submerged in the tank below the water level 125). The ozone generator 110 may receive commands from a controller 100. The ozone mitigation system may include an ozone elimination device 150 operable inside of the toilet tank 101 and configured to slow or stop the escape of ozone from the toilet tank 101. The ozone mitigation system is for mitigating the release of ozone in the ambient environment of the toilet 1100. For example, the controller 100 may cause a light to turn on for irradiating the ozone and/or a piezo transducer to turn on for reducing the ozone. Additional, different, or fewer components may be included.

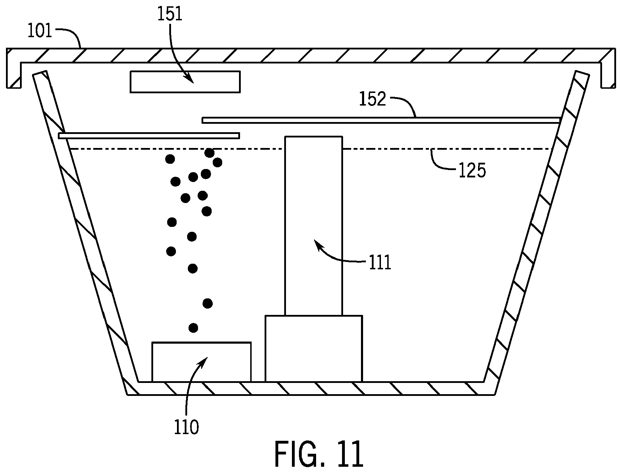

FIG. 11 illustrates an example ozone mitigation system including a light source 151 as part of the ozone elimination device 150. The light source 151 irradiates the ozone that is escaping the water in the toilet tank 101 to reduce the amount of ozone that potentially could escape the toilet tank 101. The light source 151 may be shielded from the water in the toilet tank 101 so as not to remove the ozone from the water. In some examples, the light source 151 may be replaced or combined with an ultrasound transmitter (e.g., a piezo transducer). Either source is capable of breaking down ozone into hydroxyls and/or water at the boundary of the microbubble. In some examples, the ozone is converted to oxygen has and oxygen radicals.

The light source 151 may emit a frequency or wavelength of light that is configured to eliminate ozone. For example, the light source 151 may be a UV-C lamp that produces light with a 254 nanometer wavelength. The UV-C lamp may produce light in an ozone destruction range such as 240-315 nanometer wavelength. As described herein, other wavelengths of ultraviolet light (e.g., 185 nanometer wavelength) may be harnessed to produce ozone. However, ultraviolet light at or near 254 nanometer wavelength is used here to remove ozone.

In addition, the ozone elimination device 150 may include one or more light baffles 152. The light baffles 152 shield the light from the water in the toilet tank 101 so as not to remove the ozone from the water. As the ozone leaves the water in the toilet tank 101, it travels through a path created by the light baffles 152. The ozone travels through the spaces between the light baffles 152 and eventually becomes in direct (e.g., line of sight) alignment with the light source 151. The light source 151 provides ozone eliminating light to the ozone and causes the ozone to be broken down and/or eliminated.

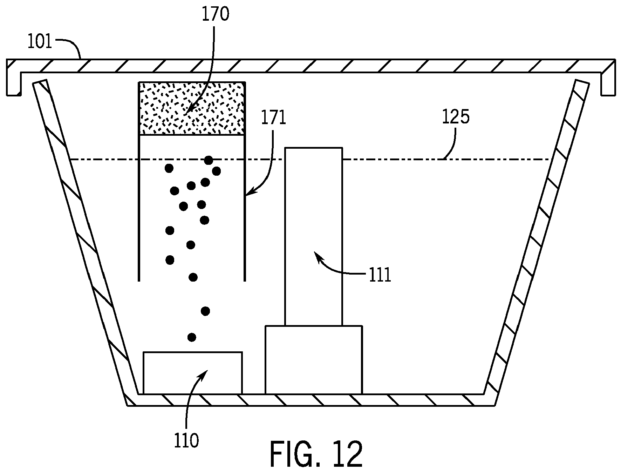

FIG. 12 illustrates an example ozone mitigation system including a filter 170 and/or a filter chamber 171 as part of the ozone elimination device 150. Additional, different, or fewer components may be included.

The filter 170 may be a carbon filter or another type of filter. The filter 170 may be surface treated to provide ozone removal characteristics. The filter chamber 171 is at least partially submerged below the water level 125 and at least partially above the water level 125. The space above the water level 125 is partially sealed from the ambient atmosphere. The filter 170 is supported within the chamber 171. The chamber 171 directs the flow of ozone that is being expelled from the water so that the ozone can be eliminated or reduced by the filter 170.

The filter 170 may be removably supported by the chamber 171. A user may remove one or more fasteners (e.g., clips, screws, snaps, or bands) to remove the filter 170 and/or install a replacement filter. The filter 170 may be replaced periodically. In some examples, the controller may provide a reminder to the user to replace the filter. In some examples, sensors may provide feedback data on the condition of the filter and the controller may determine filter replacement is recommended. For example, a sensor may detect ozone levels. When the ozone levels is greater than a threshold, or the rate of increase of ozone surpasses a threshold rate, the controller determines that the filter is no longer operating to remove ozone from the air in the tank 101. In response, the controller may display or otherwise transmit a message for filter replacement. The threshold for the ozone concentration or rate of change thereof may be set by historical data. That is, the running average of the last day, week, or month may be used as the threshold. The threshold for the ozone concentration or rate of change thereof may be set by user input.

The filter chamber 171 may be positioned at a predetermined position with respect to the ozone generator 110. The filter chamber 171 may be positioned directly above (e.g., opposite the direction of gravity) the ozone generator 110. The filter chamber 171 provides a steered air path where the ozone would be most concentrated

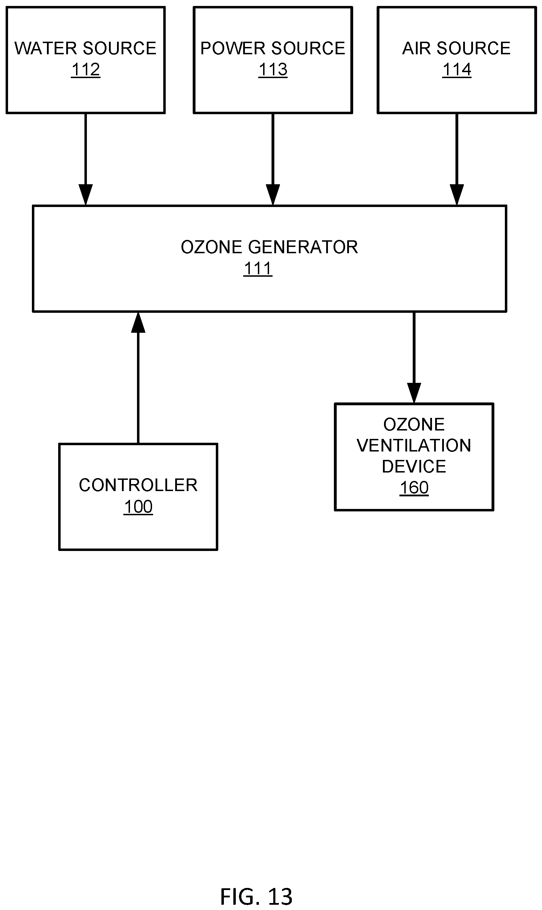

FIG. 13 illustrates an example ozone mitigation system including a ventilation device 160. The ozone mitigation system may include an ozone generator 110 including inputs comprising a power source 113 and an air source 114. The ozone generator 110 is in communication with the tank 101 (e.g., submerged in the tank below the water level 125). The ozone generator 110 may receive commands from a controller 100. The ozone mitigation system may include an ozone ventilation device 160 operable inside of the toilet tank 101 and configured control the escape of ozone from the toilet tank 101. The ozone mitigation system is for mitigating the release of ozone in the ambient environment of the toilet 1100. In this example, the ozone ventilation device 160 prevents ozone from escaping into the environment by providing a venting path for the ozone. Additional, different, or fewer components may be included.

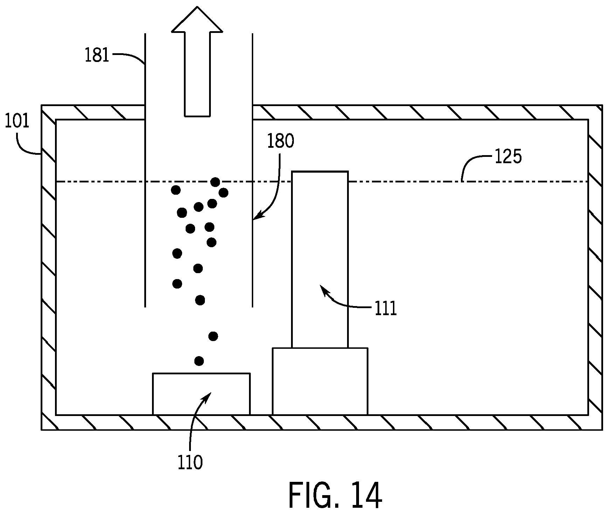

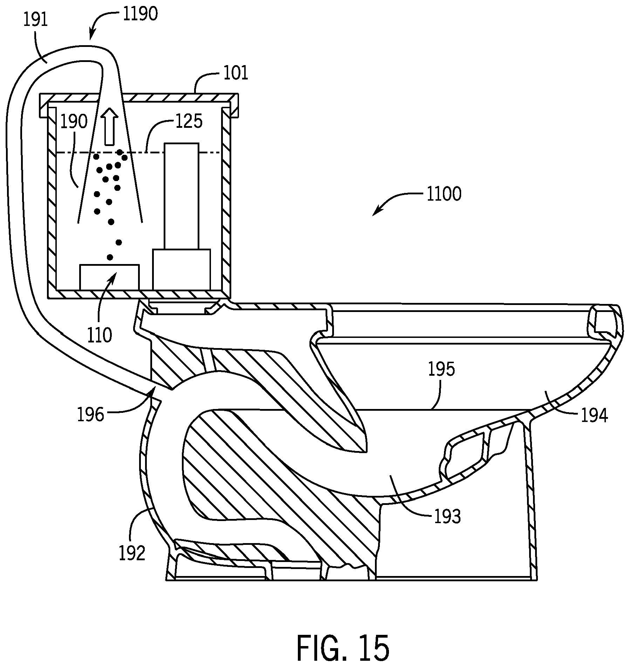

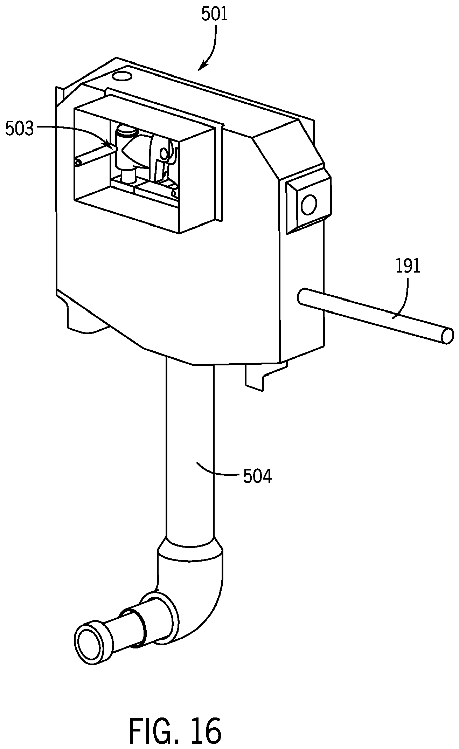

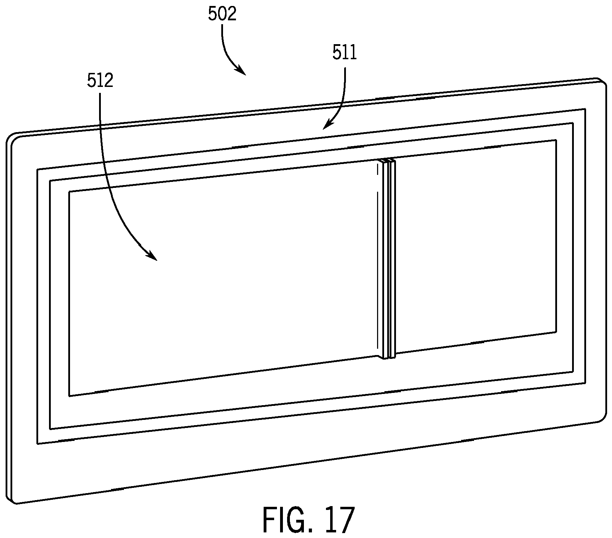

FIG. 14 illustrates an example ozone mitigation system including an external venting system for the ozone ventilation device 160. The term "external" means at least part of the path for the ozone is external to the toilet. The path of the ozone may return through another portion of the toilet (e.g., as in FIG. 15) The term "completely external" means all parts of the path for the ozone are external to the toilet (e.g., as in FIGS. 16 and 17).

The ozone ventilation device 160 may include an ozone exhaust 181 and an ozone collector 180. The ozone collector 180 includes an enclosed space under the water of the toilet tank to steer ozone into the ozone exhaust 181. The ozone collector 180 includes a conical shape or a cylindrical shape.

The ozone collector 180 may be at least partially submerged in water of the toilet tank. For example, a portion of the ozone collector 180 may be above the water level 125 and a portion of the ozone collector 180 may be below the water level 125.

The ozone collector 180 may cover a substantial portion of the tank 101. For example, the ozone collector 180 may be attached to a perimeter of the top of the toilet tank (e.g., below the lid) to cover substantially all of the tank 101 or all of the tank 101. In some examples, a vent may be included in the ozone collector 180 to vent air into the tank. That is, the ozone collector 180 may channel ozone away from the tank 101 but include a vent in the wall of the ozone collector 180 so that air can travel from the ambient environment back into the tank 101. The vent may be a release valve or a one-way valve. The vent allows the water level 125 to go down (e.g., for flushing the toilet) without creating a vacuum that could damage the ozone collector 180 or reverse the action of the ozone collector 180.

The ozone exhaust 181 is coupled to the toilet tank 101 via the ozone collector 180. The ozone exhaust 181 may be a duct, tube, or other pathway configured to carry the ozone from the tank 101 to an external location. The ozone exhaust 181 may be coupled to another room (i.e., another room separate from the location of the toilet 1100). The ozone exhaust 181 may be coupled to a building exhaust system so that ozone is expelled out of the building. The ozone exhaust 181 may include a fan in the path to help expel the ozone.

FIG. 15 illustrates an example ozone mitigation system including a sump venting system 1190 as another example ozone ventilation device 160. The sump venting system 1190 may include an ozone exhaust channel 191 and an ozone collector 190. The ozone collector 190 includes an enclosed space under the water of the toilet tank to steer ozone into the ozone exhaust. The ozone collector 190 has a conical shape such that a first opening at the bottom of the ozone collector 190 where the ozone enters the ozone collector 190 has a larger radius than a second opening at the top of the ozone collector 190 that feeds into the ozone exhaust channel 191.

When the toilet 1100 is flushed the tank 101 empties and more water is provided from the fill valve 111. As the water level rises in the tank 101, pressure is created in the space of the tank in the ozone collector 190 and or ozone exhaust channel 191 to apply a force to push the ozone through the ozone exhaust channel 191. The rising water level (or full water tank) acts to provide a seal to seal ozone from returning to the tank.

The toilet 1100 may include a bowl 194, a sump 193, and a trapway 192. Other components may be included. The sump 193 fluidly connects the bowl 194 and the trapway 192. Rim channels or other channels in the toilet 1100 may be connected to a fluid supply source, such as a household water supply, or the tank 101. Either through the channels or a jet outlet, a flow of water can be introduced to the sump 193 to, for example, to help prime a siphon in the trapway 192 to induce a flush cycle and empty the contents of the bowl 194. A water level 195 illustrates a siphon seal, that when broken induces the flush.

The trapway 192 may be coupled to the ozone exhaust channel 191. For example, the ceramics or vitreous material forming the toilet 1100 may include a port configured to be coupled with the ozone exhaust 191. The port may include an opening. The port may include a nozzle such that the ozone exhaust 191 includes a tube that fits over or inside of the nozzle. An ozone channel 196 may be formed in the toilet 1100 between the port on the outside of the toilet 1100 and the trapway 192.

The ozone exhaust 191 is configured to vent off the ozone gas that does not dissolve into the tank water. The trapway 192 may not pressurize from the buildup of ozone since it is connected to the building vent. A check valve or a water channel may be included in the ozone exhaust channel 191. The check valve or the water channel may prevent the ozone exhaust channel 191 from interfering with the flush performance (i.e. break the siphon early or prevent a siphon).

In another example, as ozone is carried by the ozone exhaust 191 to the trapway 192, additional air pressure is applied in the trapway 192. The additional air pressure applied a downward force within the trapway 192 on the water level 195. The downward force helps to break the siphon and cause the toilet 1100 to flush. In addition, as the siphon is broken and immediately after, the ozone escape through the trapway. The ozone escapes from the trapway to a sewer line, a septic system, or another passage external to the toilet 1100.

FIG. 16 illustrates an example in-wall tank 501 including the ozone mitigation system. The in-wall tank 501 may include any of the components of the embodiments described herein. The in-wall tank 501 is coupled to a toilet (e.g., toilet pedestal) via a water pipe 504. An actuator 502, as illustrated by FIG. 17, may be mounted on a wall and coupled to the in-wall tank 501 for trigger a flush of the toilet via a user input (e.g., pressure).

The in-wall tank 501 may include any of the ozone flow restrictor 120 examples, any of the circulation device 130 examples, any of the ozone elimination device 150 examples, or any of the ozone collection devices described herein. In addition, as illustrated, the in-wall tank 501 includes an example ozone ventilation device 160 as ozone exhaust 191. The ozone exhaust 191 receives the ozone generated within the in-wall tank 501 and provides it to an external location. The ozone exhaust 191 may vent the ozone to outside of the building, a sewer pipe, or a septic system. In addition, the ozone exhaust 191 may transfer the ozone to a chamber where it is combusted or otherwise destroyed.

FIG. 17 illustrates an example seal 511 for a flush control 512 on the actuator 502 for an the in-wall tank 501. The seal 511 may be shaped to fit opening 503 in the in-wall tank 501. The seal 511 may be configured to seal the opening 503 to prevent ozone from leaking around the flush control 512 out of the in-wall tank 501 where the ozone is generated and dissolved in the water that is used to flush the toilet.

In any of the examples herein, a light (e.g., UV-C) may be included in the bowl or in the proximity of the bowl, for example coupled to a toilet seat or a toilet lid. The light may additionally break down ozone that escapes through the bowl, for example, through trapway 192 or otherwise.

FIG. 18 illustrates an example controller for any off the ozone mitigation systems. The controller 400 may include a processor 300, a memory 352, and a communication interface 353 for interfacing with devices or to the internet and/or other networks 346. In addition to the communication interface 353, a sensor interface may be configured to receive data from the sensors described herein or data from any source for analyzing, ozone, air, or water properties or the operation of the appliances described herein. The components of the control system 400 may communicate using bus 348. The control system 400 may be connected to a workstation or another external device (e.g., control panel) and/or a database for receiving user inputs, system characteristics, and any of the values described herein.

Optionally, the control system 400 may include an input device 355 and/or a sensing circuit in communication with any of the sensors. The sensing circuit receives sensor measurements from as described above. The input device 355 may include a switch (e.g., actuator), a touchscreen coupled to or integrated with, a keyboard, a remote, a microphone for voice inputs, a camera for gesture inputs, and/or another mechanism.

Optionally, the control system 400 may include a drive unit 340 for receiving and reading non-transitory computer media 341 having instructions 342. Additional, different, or fewer components may be included. The processor 300 is configured to perform instructions 342 stored in memory 352 for executing the algorithms described herein. A display 350 may be supported by any of the components described herein. The display 350 may be combined with the user input device 355.

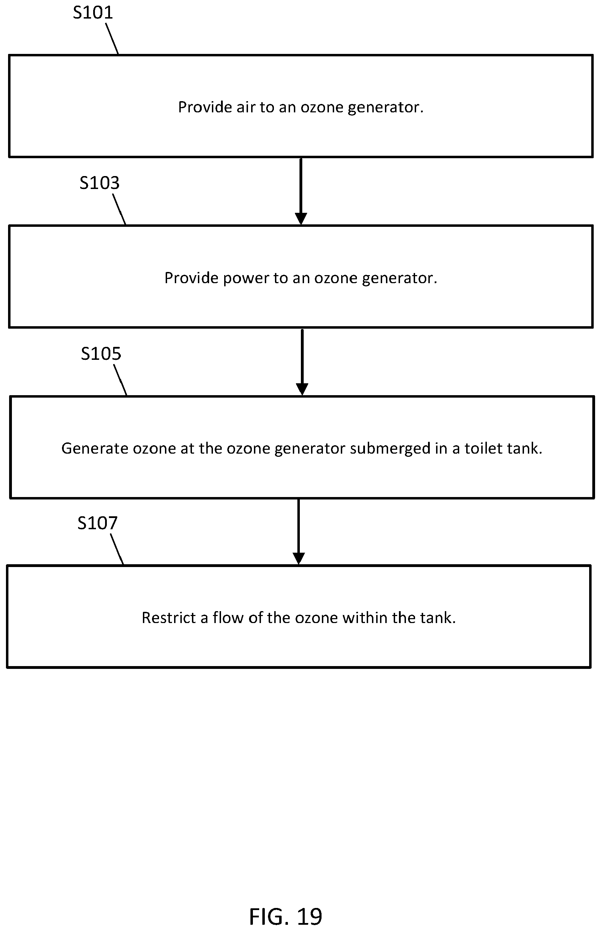

FIG. 19 illustrates a flow chart for the controller of FIG. 18. The acts of the flow chart may be performed by any combination of the control system 400, the network device, or the server. Additional, different or fewer acts may be included.

Acts S101 and S103 may be alternatives. Acts S101 and S103 may be performed together at substantially the same time. At act S101, the controller 400 may send a signal to a valve to open an air path and provide air to the ozone generator 110. At act S103, the controller 400 may turn on a power supply to provide electrical power to the ozone generator 110. For example, the controller 400 may turn on the ozone generator 110 and prepare the ozone generate 110 to generate ozone.

At act S105, the controller 400 may send a signal (e.g., a command) to the ozone generator 110 to generate ozone in a toilet tank in response to the air supply or power supply. The signal may turn on a relay or other switch to activate a corona charge in the ozone generator 110.

At act S107, the ozone produced by the ozone generator 110 is restricted. In some examples, this occurs passively based on structures that limit the rising bubbles of ozone. In some examples, this occurs actively, and the controller 400 may turn on an activation signal for the ozone restrictor as a fan, an impeller, a pump, a blade or another device. The controller 400 may generate a shutoff command to subsequently deactivate the ozone flow restrictor. The shutoff command may turn off the fan, the impeller, the pump, the blade, or the other device. The shutoff command may be issued based on a timer (e.g., part of a flush cycle), in response to user input, or in response to sensor data.

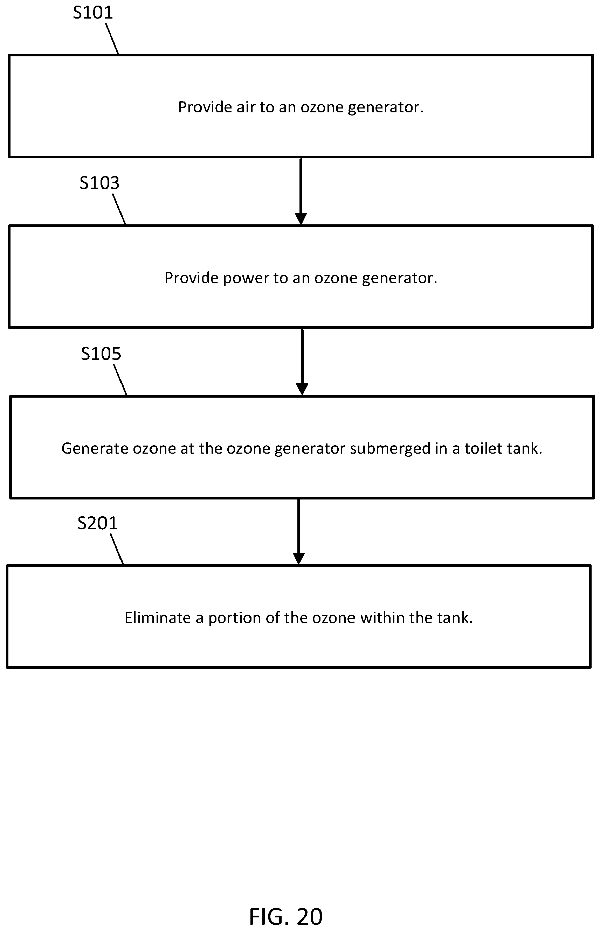

FIG. 20 illustrates a flow chart for the controller of FIG. 18. The acts of the flow chart may be performed by any combination of the control system 400, the network device, or the server. Additional, different or fewer acts may be included.

In the example illustrated, FIG. 20 includes act S201 as an alternative to act S107. In act S201, the controller 400 generates a command for an ozone elimination device. The elimination command may turn on a valve that diverts ozone to a destruction chamber. The elimination command may turn on a light (e.g., UV-C light) that is configured to break down ozone by irradiating ozone expelled from the water of the toilet tank. In other examples, the elimination command provides a pathway for filtering ozone expelled from the water of the toilet tank.

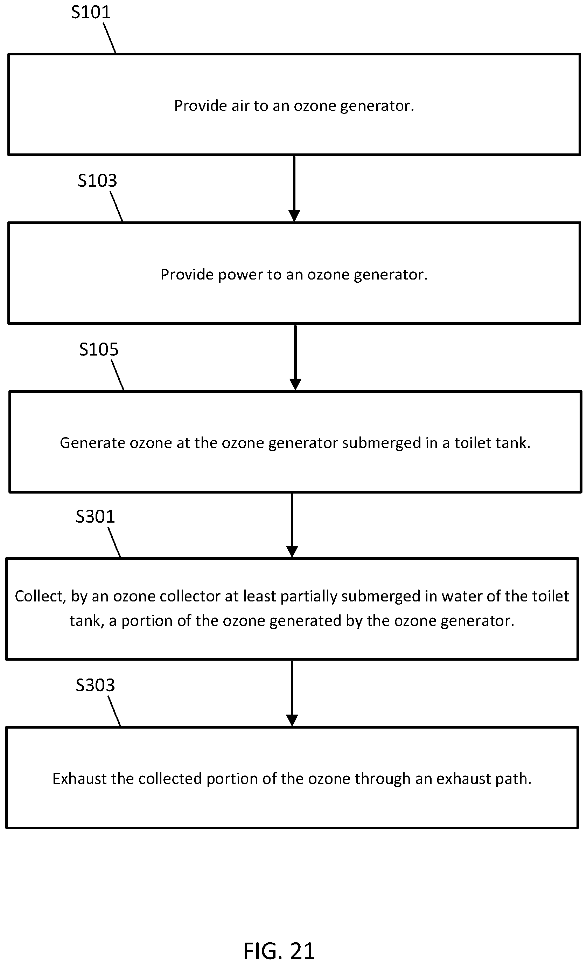

FIG. 21 illustrates a flow chart for the controller of FIG. 18. The acts of the flow chart may be performed by any combination of the control system 400, the network device, or the server. Additional, different or fewer acts may be included. In the example illustrated, FIG. 20 includes acts S301 and S303 as an alternative to act S107. In act S301, the exhaust mitigation system collects ozone at an ozone collector that is at least partially submerged in the water of the toilet tank. The ozone collector may be opened via a valve or other port. The controller 400 may generate a motor command or valve command to open the ozone collector. In act S303, an exhaust path provides a path for the ozone to another chamber external to the toilet tank. The controller 400 may open a wastegate or other door to provide the path through the exhaust path.

Processor 300 may be a general purpose or specific purpose processor, an application specific integrated circuit (ASIC), one or more programmable logic controllers (PLCs), one or more field programmable gate arrays (FPGAs), a group of processing components, or other suitable processing components. Processor 300 is configured to execute computer code or instructions stored in memory 352 or received from other computer readable media (e.g., embedded flash memory, local hard disk storage, local ROM, network storage, a remote server, etc.). The processor 300 may be a single device or combinations of devices, such as associated with a network, distributed processing, or cloud computing.

Memory 352 may include one or more devices (e.g., memory units, memory devices, storage devices, etc.) for storing data and/or computer code for completing and/or facilitating the various processes described in the present disclosure. Memory 352 may include random access memory (RAM), read-only memory (ROM), hard drive storage, temporary storage, non-volatile memory, flash memory, optical memory, or any other suitable memory for storing software objects and/or computer instructions. Memory 352 may include database components, object code components, script components, or any other type of information structure for supporting the various activities and information structures described in the present disclosure. Memory 352 may be communicably connected to processor 300 via a processing circuit and may include computer code for executing (e.g., by processor 300) one or more processes described herein. For example, memory 298 may include graphics, web pages, HTML files, XML files, script code, shower configuration files, or other resources for use in generating graphical user interfaces for display and/or for use in interpreting user interface inputs to make command, control, or communication decisions.

In addition to ingress ports and egress ports, the communication interface 353 may include any operable connection. An operable connection may be one in which signals, physical communications, and/or logical communications may be sent and/or received. An operable connection may include a physical interface, an electrical interface, and/or a data interface. The communication interface 353 may be connected to a network. The network may include wired networks (e.g., Ethernet), wireless networks, or combinations thereof. The wireless network may be a cellular telephone network, an 802.11, 802.16, 802.20, or WiMax network, a Bluetooth pairing of devices, or a Bluetooth mesh network. Further, the network may be a public network, such as the Internet, a private network, such as an intranet, or combinations thereof, and may utilize a variety of networking protocols now available or later developed including, but not limited to TCP/IP based networking protocols.

While the computer-readable medium (e.g., memory 352) is shown to be a single medium, the term "computer-readable medium" includes a single medium or multiple media, such as a centralized or distributed database, and/or associated caches and servers that store one or more sets of instructions. The term "computer-readable medium" shall also include any medium that is capable of storing, encoding, or carrying a set of instructions for execution by a processor or that cause a computer system to perform any one or more of the methods or operations disclosed herein.

In a particular non-limiting, exemplary embodiment, the computer-readable medium can include a solid-state memory such as a memory card or other package that houses one or more non-volatile read-only memories. Further, the computer-readable medium can be a random access memory or other volatile re-writable memory. Additionally, the computer-readable medium can include a magneto-optical or optical medium, such as a disk or tapes or other storage device to capture carrier wave signals such as a signal communicated over a transmission medium. A digital file attachment to an e-mail or other self-contained information archive or set of archives may be considered a distribution medium that is a tangible storage medium. Accordingly, the disclosure is considered to include any one or more of a computer-readable medium or a distribution medium and other equivalents and successor media, in which data or instructions may be stored. The computer-readable medium may be non-transitory, which includes all tangible computer-readable media.

In an alternative embodiment, dedicated hardware implementations, such as application specific integrated circuits, programmable logic arrays and other hardware devices, can be constructed to implement one or more of the methods described herein. Applications that may include the apparatus and systems of various embodiments can broadly include a variety of electronic and computer systems. One or more embodiments described herein may implement functions using two or more specific interconnected hardware modules or devices with related control and data signals that can be communicated between and through the modules, or as portions of an application-specific integrated circuit. Accordingly, the present system encompasses software, firmware, and hardware implementations.

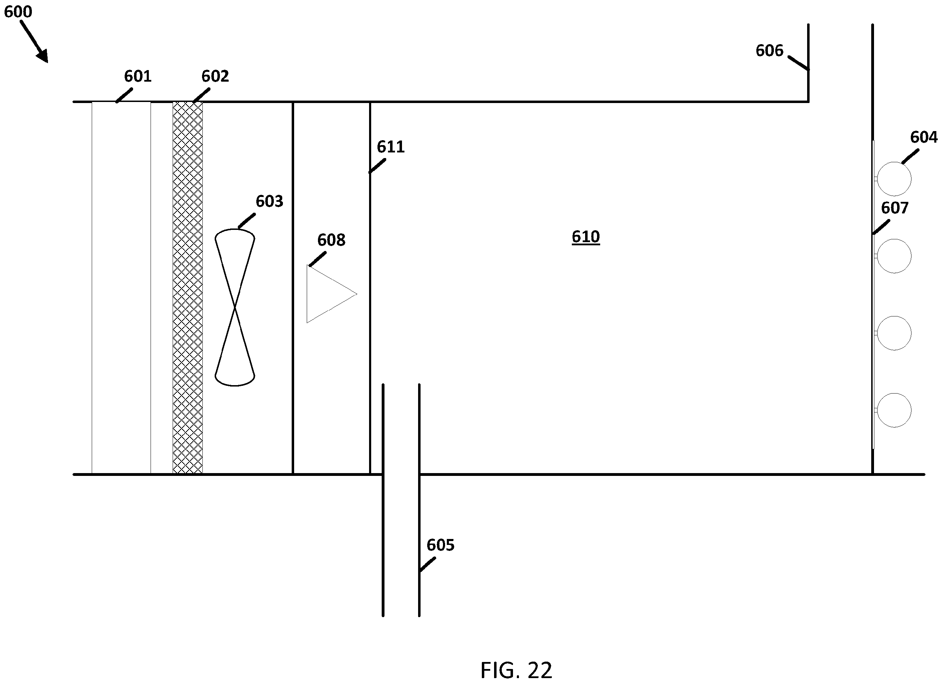

FIG. 22 illustrates another ozone mitigation device 600 for a plumbing fixture. The plumbing fixture may be a pipe or an appliance include a flow of water such as bathtubs, bidets, drinking fountains, hose bibs, sinks, showers, faucets, shower heads, urinals, or other devices. The plumbing fixture may include water source or input and water drain or output. The ozone generation and mitigation systems described herein may be placed within the plumbing fixture or adjacent to the plumbing fixture and coupled to the plumbing fixture.

The example of FIG. 22 includes an ozone mitigation device 600 including at least an ozone generator 608, a cavity 610 or other tank, and a light 604. The cavity 610 includes at least an input port 605 and an output port 606. The ozone mitigation device 600 may be installed "in-line" along any plumbing system in which an upstream water source is connected to the input port 605 and the drain, or other plumbing fixture, is connected to the output port 606. As illustrated one or more additional components may include an air source or air input 601, a filter 602, a fan 603, and a partially transparent window 607. Additional, different, or fewer components may be included.

The ozone generator 608 may include any of the example ozone devices described herein, including a corona charger. For example, a corona charger may be configured to accumulate electric charge from a power source and apply the electric charge to air from an air source. An electrical conductor, such as a corona discharge tube or an ozone plate, may receive a high voltage may an output an electrical discharge caused by the ionization of air surrounding the conductor carrying the high voltage.

The ozone generator 608 may receive commands from a controller 100. For example, the controller 100 may send a command to turn on the power source and/or connect the power source to the corona charger at a predetermined timing. In some examples, the controller 100 is configured to cause a predetermined amount of ozone to be generated in the cavity 610. For example, the cavity 610 may include an ozone sensor that provides feedback to the controller 100, and the controller 100 generates a command for the corona charger in response to the feedback. The controller 100 may turn on the ozone generator 608 when the ozone sensor indicates that the ozone level has fallen below a minimum level and/or the controller 100 may turn off the ozone generator 608 when the ozone sensor indicates that the ozone level has gone above a maximum level.

The ozone generator 608 is in communication with the plumbing fixture and configured to inject ozone into a flow of water through the plumbing fixture. The flow of water is disinfected by the ozone.

The air input 601 may be an air intake. For example, the air input 601 may include a vent or an aperture configured to take in air at the ozone mitigation device 600. The flow of air may be driven by fan 603 including one or more blades for drawing air into the ozone mitigation device 600. The controller 100 may sending commands (e.g., start command, stop command, duty cycle) to the fan 603.

In some examples, between the fan 603 and the air input 601 is a filter 602. The filter 602 may include a screen (e.g., metal or plastic grid with holes), a network of fibers, or paper configured to remove particles from the flow of air. In addition or in the alternative, a filter may be included in the water.

In some examples, a membrane 611 may provide the air to water junction between the ozone generator 608 and the cavity 610. The membrane 611 may be permeable to air but not to water. Thus, the air from the ozone generator 608 is allowed to flow through the membrane 611 into the cavity 610. However, water in the cavity 610 cannot travel in the opposite direction through the membrane 611.

The light 604 may be a variety of types of light sources such as an ultraviolet (UV) light. The UV light may have a predetermined frequency or wavelength, which may be a range of wavelengths or frequencies for the light emitted from the light 604. The predetermined frequency or wavelength may be configured to dissipate the ozone in the flow of water. The controller 100 may send commands to the light 604 to start the light 604 or stop the light 604. The controller 100 may send commands to the light 604 to set the wavelength of the light 604.

The light 604 may perform both disinfection and ozone dissipation. For example, the light 604 may include ultra-violet germicidal irradiation (UVGI) to kill microbes in the water. The germicidal irradiation may be optimized by a wavelength band of 200 to 280 nanometers (nm). The ozone dissipation may be optimized by a wavelength band of 240 to 315 nm. Thus, the light 604 may be tuned to an overlapping wavelength band of 240 to 280 nm to perform both the germicidal irradiation and the ozone dissipation.

A specific wavelength may be selected based on the structure or gas of an arc lamp or light emitting diode (LED) that is available in the overlapping wavelength band. One example is a low-pressure mercury vapor art lamp at approximately 254 nm.

The light 604 may include multiple light sources arranged radially or vertically along at least one side of the cavity 610. The side of the cavity 610 may include a partially transparent window 607. The window 607 may be formed of glass or plastic and allow the predetermined wavelength of the light source 604 to travel into the cavity 610 and dissipate the ozone in the water.

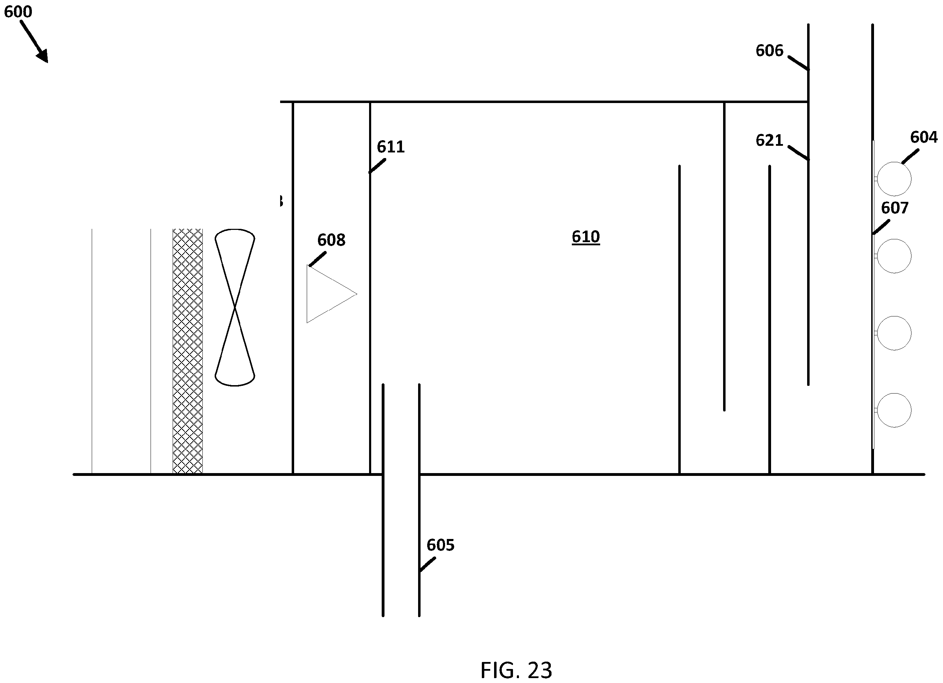

FIG. 23 illustrates another ozone mitigation device including an ozone dissipation path. The ozone dissipation path may provide a path for the water to flow in proximity of the light source 604 in order to increase the ozone dissipation provided by the light source 604.

In one embodiment, one or more baffles 621 direct the flow of water in a particular pattern than increases the exposure of the water to the light source 604 to maximize the dissipation of ozone. For example, as shown in FIG. 23 the baffles 621 may be arranged to provide a path with multiple sections in opposite direction so that the flow of water moves in a snaking pattern (e.g., back and forth) across the path of the light from the light source 604. Alternately, the baffles 621 may be arranged radially to create spirals for the flow of water. The baffles 621 may be arranged to create a serpentine path. In any of these examples, the one or more baffles 621 cause the flow of water to travel in a first direction upstream of the baffle and in a second direction downstream of the baffle.

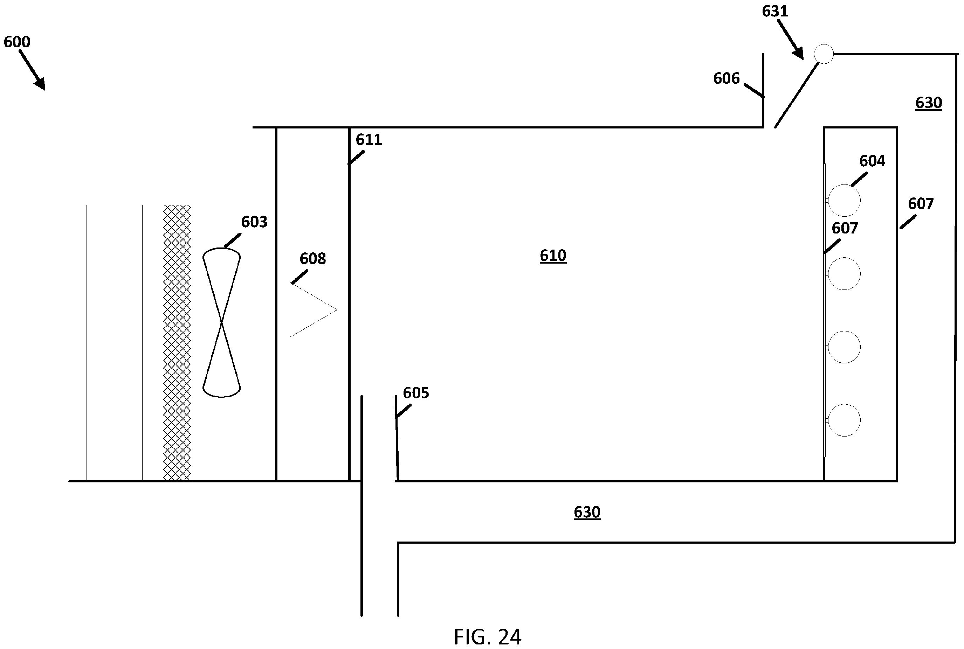

FIG. 24 illustrates another ozone mitigation device with a bypass path 630 in which at least a portion of the bypass path is in proximity to the light source. A valve 631 may be configured to open or close the bypass path 630 downstream of the ozone generator 608. For example, the valve 631 may include a solenoid or a stepper motor that moves a valve plate to open and close the output 606 of the cavity 610. When the valve 631 is open, the output 606 empties the cavity 610 normally as more water enters the cavity from the input 605. When the valve 631 is closed, the bypass path 630 is created so that the flow of water recirculates in the cavity 610. That is, the water (e.g., the same water droplets or molecules) travel once through the cavity 610, then through the bypass path 630, then through the cavity 610 a second time. Multiple paths through the cavity 610 increases the exposure of the flow of water to the light source 604 and increases the dissipation of ozone in the flow of water. The bypass path 630 may join with the input 605 upstream of the cavity 610.

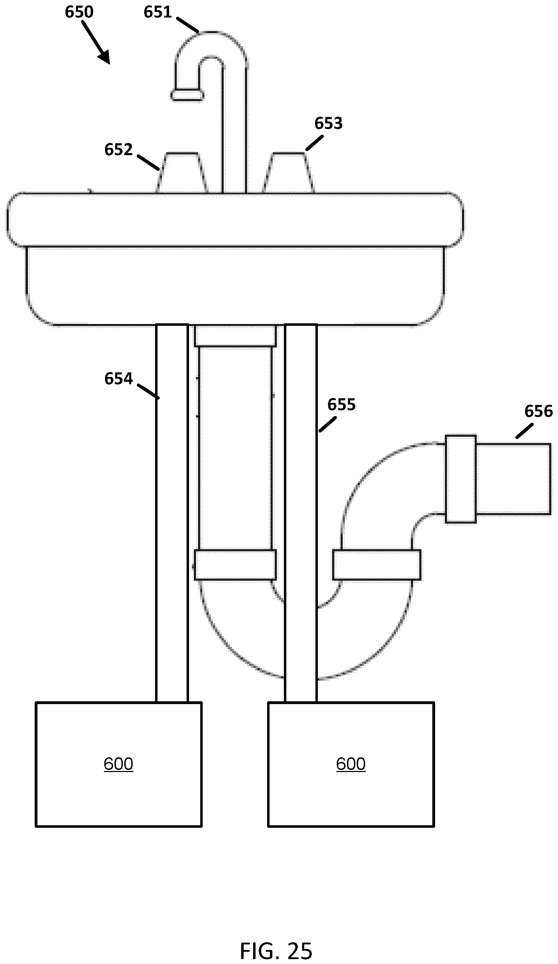

FIG. 25 illustrates a sink 650 with an example ozone mitigation path. The sink 650 includes a basin and a faucet including a lever 651 and one or more handles 652, 653 to operate the faucet. A first handle 652 is connected to a hot water supply pipe 654 including a first plumbing fixture including the ozone mitigation system 600 including the components of the ozone mitigation system of any one or more of FIGS. 22-24. A second handle 653 is connected to a cold water supply pipe 655 including a second plumbing fixture having the ozone mitigation system 600 including the components of the ozone mitigation system of any one or more of FIGS. 22-24.

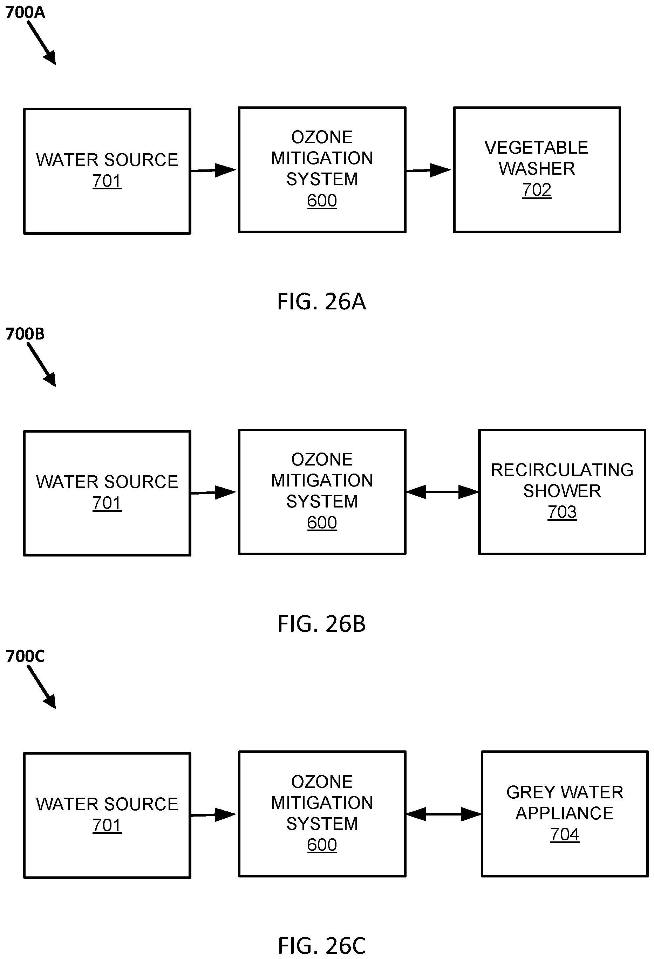

FIG. 26A-C illustrate example appliances for use with an ozone mitigation system including a recirculating shower, a vegetable washer, a grey water recycling device, and additional examples may include a drinking fountain, a water bottle filler, a bathtub, and a soda machine.

FIG. 26A includes a water source 701, the ozone mitigation system 600, and a vegetable washer 702. In one embodiment, the vegetable washer 702 may include a tray for lowering vegetables, fruit, or other food items into the cavity 610 of the ozone mitigation system 600. The ozone in the cavity 610 washes contaminants from the food items. The ozonated water disinfects the food items. After the food items are washed, and optionally removed from the cavity 610, the light source 604 is activated to at least partially remove the ozone from the water. The water is then released from the cavity 610 and utilized for another purpose or retained in the cavity 610 until the vegetable washer 702 is used again.

FIG. 26B includes a water source 701, the ozone mitigation system 600, and a recirculating shower system 703. The recirculating shower system 703 may include at least one shower outlet, a shower panel fluidly coupled to the shower outlet, a shower tray, and a soap and debris management assembly. The shower panel may be configured to house water recirculation equipment within a showering space (e.g., a corner). In various embodiments, the shower panel may facilitate access to shower control components for ease of installation and/or servicing. The shower panel may also be designed to serve as an aesthetic cover to obscure potentially unsightly shower control components. In various embodiments, the shower control components may include, but are not limited to one or more pumps, motors, filters, etc. In various embodiments, the shower panel may be removably coupled to the region within the shower via one or more hinges or latches.

Water that exits the recirculating shower 703 may be provided to the ozone mitigation system 600 where it is ozonated for the purposes of disinfection, for example, by the ozone generator 608, and then subsequently irradiated by light source 604 for removal of the ozone from the water. The water, after disinfection and ozone mitigation, may be returns to the recirculating shower 703. Upstream of the ozone mitigation system 600, or incorporated into the ozone mitigation system 600, for example, by the filter 602, the soap and debris management assembly may provide an initial stage of removing larger particles from the spent shower water.

FIG. 26C includes a water source 701, the ozone mitigation system 600, and a grey water appliance 704. The grey water appliance 704 may be a water consuming appliance that either provides water to a grey water system or is fed water by the grey water system. Water output from the grey water appliance 704 flows into the ozone mitigation system 600 where the ozone generator 608 adds ozone to the water. Optionally, the filter 602 may first filter out contaminants from the water.

The ozonated recycled water travels to the proximity of the light source 604 where the ultraviolet light dissipated or otherwise aids in the removal of the ozone from the water. The output off the ozone mitigation system 600 may provide the water to another grey water appliance.

In a first embodiment, the multi-stage water recycling system may include a first set of water consuming appliances that consumes fresh water and outputs a first tier of greywater, a first ozone mitigation system 600 that treats the first tier of greywater and outputs a treated first tier of greywater, a second set of water consuming appliances that consumes the treated first tier of greywater and outputs a second tier of greywater, a second ozone mitigation system 600 that treats the second tier of greywater and outputs a treated second tier of greywater, and a third set of water consuming appliances that consumes the treated second tier of greywater.

In the first embodiment, the first set of water consuming appliances include at least one potable water appliance such as a faucet, a water cooler, a refrigerator, or an icemaker. The second set of water consuming appliances include at least one bathing appliance. The bathing appliance may include a bathtub or a shower. The third set of water consuming appliances includes a toilet.

In a second embodiment, the multi-stage water recycling system includes a first set of water consuming appliances that consumes fresh water and outputs a first tier of greywater, a first ozone mitigation system 600 that treats the first tier of greywater and outputs a treated second tier of greywater, a second ozone mitigation system 600 that treats a first portion of the treated second tier of greywater to a first tier of greywater, a second set of water consuming appliances that consumes a second portion of the treated second tier of greywater and outputs wastewater, and a third set of water consuming appliances that consumes a portion of the first tier of greywater. A portion of the treated first tier of greywater from the second ozone mitigation system 600 is provided to the first ozone mitigation system 600.

In the second embodiment, the first set of water consuming appliances include at least one potable water device, the second set of water consuming appliances includes a toilet, and the third set of water consuming appliances includes at least one bathing appliance.

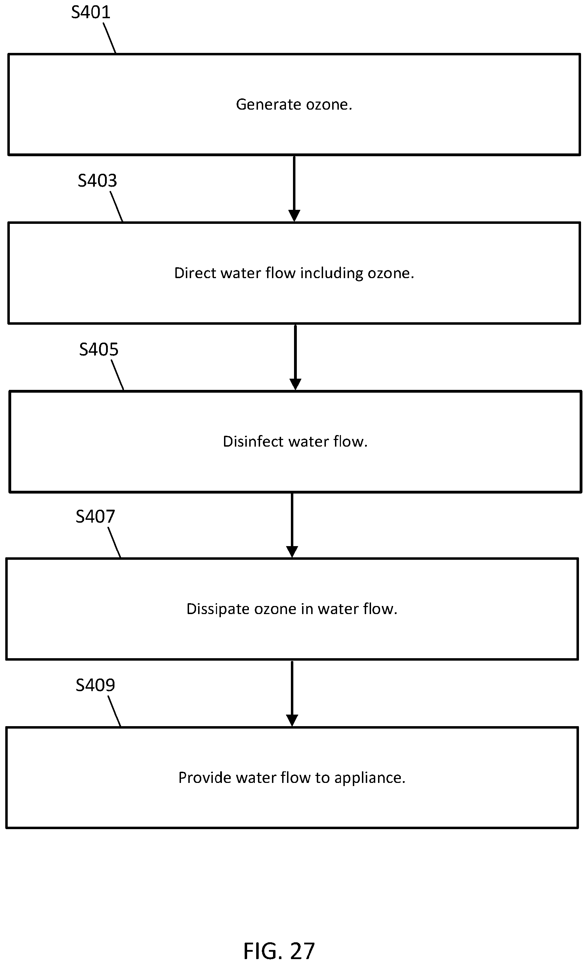

FIG. 27 illustrates another example flow chart for an ozone mitigation device 600. Additional, different, or fewer acts may be included.

At act S401, the method includes generating ozone at a plumbing fixture including a flow of water. The ozone may be generated by turning on the ozone generator 608. For example, the controller 100 may turn on the ozone generator 608 based on feedback from an ozone sensor to maintain a predetermine ozone level or range for the ozone level. The controller 100 may turn on the ozone generator 608 according to a predetermined schedule or time interval. For example, the ozone generator 608 may be operated at a certain time of day or every hour.

At act S403, the method includes directing the water flow including the ozone into a separate chamber or location within a chamber. The water flow may be directed in a circulation pattern within the chamber. The water flow may be directed in a circuitous path to increase the time the ozone is in the chamber. The water flow may be directed into a recirculation path or bypass path to traverse the chamber multiple times.

At act S405, the method includes disinfecting the water flow via the ozone. The ozone may disinfect the water through contact with the water.

At act S407, the method includes dissipating or removing at least a portion of the ozone in the water flow via a light source (e.g., ultraviolet light within a predetermined frequency range).

At act S409, the method includes providing the water flow to appliance. The appliance may be a toilet, a shower, a sink, a urinal, a faucet, a bidet, or another device that consumes water. The water flow may be provided directly (e.g., via a pipe) from the chamber to the appliance. The water flow may be provided indirectly from the chamber to the appliance. For example, the water may be pumped from the chamber to a tank (e.g., grey water tank) and then pumped to the appliance.

The illustrations of the embodiments described herein are intended to provide a general understanding of the structure of the various embodiments. The illustrations are not intended to serve as a complete description of all of the elements and features of apparatus and systems that utilize the structures or methods described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive.

While this specification contains many specifics, these should not be construed as limitations on the scope of the invention or of what may be claimed, but rather as descriptions of features specific to particular embodiments of the invention. Certain features that are described in this specification in the context of separate embodiments can also be implemented in combination in a single embodiment. Conversely, various features that are described in the context of a single embodiment can also be implemented in multiple embodiments separately or in any suitable sub-combination. Moreover, although features may be described above as acting in certain combinations and even initially claimed as such, one or more features from a claimed combination can in some cases be excised from the combination, and the claimed combination may be directed to a sub-combination or variation of a sub-combination.

One or more embodiments of the disclosure may be referred to herein, individually and/or collectively, by the term "invention" merely for convenience and without intending to voluntarily limit the scope of this application to any particular invention or inventive concept. Moreover, although specific embodiments have been illustrated and described herein, it should be appreciated that any subsequent arrangement designed to achieve the same or similar purpose may be substituted for the specific embodiments shown. This disclosure is intended to cover any and all subsequent adaptations or variations of various embodiments. Combinations of the above embodiments, and other embodiments not specifically described herein, will be apparent to those of skill in the art upon reviewing the description.

It is intended that the foregoing detailed description be regarded as illustrative rather than limiting and that it is understood that the following claims including all equivalents are intended to define the scope of the invention. The claims should not be read as limited to the described order or elements unless stated to that effect. Therefore, all embodiments that come within the scope and spirit of the following claims and equivalents thereto are claimed as the invention.

A first aspect provides an ozone mitigation system for a toilet, the ozone mitigation system comprising:
an ozone generator in communication with a toilet tank;
an air source connected to the ozone generator;
a power source connected to the ozone generator; and
an ozone flow restrictor operable inside of the toilet tank and configured to modify a flow of ozone from the ozone generator through the toilet tank.

The ozone mitigation system may comprise:
a corona charger configured to accumulate electric charge from the power source and apply the electric charge to air from the air source.

The ozone flow restrictor may comprise:
at least one obstacle arranged in a path of the flow of ozone.

The at least one obstacle may include a bead column or a ring column.

The at least one obstacle may include one or more baffles.

The at least one obstacle may include a rotating element.

The ozone flow restrictor may comprise:
an impellor configured to propel ozone through the toilet tank using one or more fins.

The impellor may propel the ozone at an angle to the flow of ozone rising from the toilet tank.

The ozone flow restrictor may comprise:
a pump configured to create a recirculation path for the ozone within the toilet tank.

The recirculation path may be substantially vertical.

The ozone flow restrictor may comprise:
a chamber enclosing a space above a predetermined water level in the toilet tank, wherein the recirculation path passes through the recirculation path.

The ozone flow restrictor may comprise:
an extended water tube for extending a path of the flow of ozone.

The ozone flow restrictor may comprise:
a fill valve assembly configured to draw ozone from the ozone generator and expel the ozone to the toilet tank at a predetermined position.

The fill valve assembly may comprise:
a water inlet channel connected to a line supply of water; and
an ozone inlet channel connected to the water inlet via a venturi opening.

The ozone mitigation system may comprise:
a filter configured to slow the flow of ozone from the fill valve assembly.

The predetermined position may be at a bottom of the toilet tank.

A second aspect provides a method for ozone mitigation in a toilet tank, the method comprising:
providing air to an ozone generator;
providing power to the ozone generator;
generating, at the ozone generator, ozone in a toilet tank in response to the air or the power; and
restricting a flow of the ozone in the toilet tank.

The ozone generator may be submerged in water of the toilet tank.

The method may comprise:
providing power to an ozone flow restrictor to restrict the flow of the ozone in the toilet tank.

The method may comprise:
deactivating the ozone flow restrictor in response to a command.

A third aspect provides an ozone mitigation system for a toilet, the ozone mitigation system comprising:
an air supply;
a power supply;
an ozone generator configured to generate ozone, in a toilet tank, from the air supply and the power supply; and
an ozone eliminator configured to reduce ozone expelled from the toilet tank.

The ozone mitigation system may comprise:
an ozone flow restrictor operable inside of the toilet tank and configured to modify a flow of ozone from the ozone generator through the toilet tank.

The ozone eliminator may include a light source within the toilet tank.

The ozone eliminator may include a filter within the toilet tank.

The ozone mitigation system may comprise:
a controller configured to generate an activation command for the ozone eliminator.

The activation command may be generated in response to a user input.

The activation command may be generated in sensor data.

A fourth aspect provides a method of ozone mitigation for a toilet tank, the method comprising:
providing air to an ozone generator;
providing power to the ozone generator;
generating, at the ozone generator, ozone in the toilet tank in response to the air or the power; and
eliminating at least a portion of ozone expelled from water of the toilet tank.

Eliminating the at least the portion of the ozone may comprise:
irradiating ozone expelled from the water of the toilet tank.

Eliminating the at least the portion of the ozone may comprise:
filtering ozone expelled from the water of the toilet tank.

A fifth aspect provides an ozone mitigation system for a toilet, the ozone mitigation system comprising:
an air input to a toilet tank;
an ozone generator configured to generate ozone, in the toilet tank, from the air input;
an ozone collector at least partially submerged in water of the toilet tank; and
an ozone exhaust coupled to the toilet tank.

The ozone collector may be submerged in the water of the toilet tank when the water of the toilet tank is at a full level.

The ozone collector may include an enclosed space under the water of the toilet tank to steer ozone into the ozone exhaust.

The ozone collector may include a conical shape or a cylindrical shape.

The ozone exhaust may be coupled to a building exhaust system.

The ozone mitigation system may comprise:
a valve or gate to open and close the ozone exhaust.

The ozone mitigation system may comprise:
a controller configured to generate a command for the valve or gate to open and close the ozone exhaust.

A sixth aspect provides a toilet comprising:
the ozone mitigation system of the firth aspect;
a bowl; and
a trapway coupled to the ozone exhaust.

The ozone exhaust may provide air pressure to assist in flush of the bowl.

A seventh aspect provides a toilet comprising:
the ozone mitigation system of the fifth aspect;
a bowl; and
an in-wall tank coupled to the ozone exhaust.

An eighth aspect provides a method of ozone mitigation for a toilet tank, the method comprising:
providing air to an ozone generator;
providing power to the ozone generator;
generating, at the ozone generator, ozone in a toilet tank in response to the air or the power;
collecting, by an ozone collector at least partially submerged in water of the toilet tank, a portion of the ozone generated by the ozone generator; and
exhausting the collected portion of the ozone through an exhaust path.

A ninth aspect provides an ozone mitigation system for a plumbing fixture, the ozone mitigation system comprising:
an ozone generator in communication with the plumbing fixture and configured to inject ozone into a flow of water through the plumbing fixture, wherein the flow of water is disinfected by the ozone; and
a light source configured to dissipate the ozone in the flow of water.

The ozone mitigation system may comprise:
an air source connected to the ozone generator; and
a power source connected to the ozone generator.

The ozone generator may include a corona charger coupled to the power source and configured to generate the ozone.

The ozone mitigation system may comprise:
a filter upstream of the ozone generator and configured to remove one or more particles from the air source.

The ozone mitigation system may comprise:
a baffle configured to direct the flow of water in proximity to the light source.

The flow of water may travel in a first direction upstream of the baffle and in a second direction downstream of the baffle.

The ozone mitigation system may further comprise:
a valve configured to open or close a bypass path downstream of the ozone generator.

At least a portion of the bypass path may be in proximity to the light source.

A tenth aspect provides a method for ozone mitigation, the method comprising:
generating ozone at a plumbing fixture including a flow of water;
disinfecting the water flow via the ozone;
dissipating the ozone in the water flow via a light source; and
providing the water flow to appliance.

## Claims

1. An ozone mitigation system for a toilet, the ozone mitigation system comprising:
an ozone generator in communication with a toilet tank;
an air source connected to the ozone generator;
a power source connected to the ozone generator; and
an ozone flow restrictor operable inside of the toilet tank and configured to modify a flow of ozone from the ozone generator through the toilet tank.

2. The ozone mitigation system of claim 1, further comprising:
a corona charger configured to accumulate electric charge from the power source and apply the electric charge to air from the air source.

3. The ozone mitigation system of claim 1 or claim 2, wherein the ozone flow restrictor comprises:
at least one obstacle arranged in a path of the flow of ozone.

4. The ozone mitigation system of claim 3, wherein the at least one obstacle includes a bead column or a ring column and/or wherein the at least one obstacle includes one or more baffles and/or wherein the at least one obstacle includes a rotating element.

5. The ozone mitigation system of any one of claims 1 to 4, wherein the ozone flow restrictor comprises:
an impellor configured to propel ozone through the toilet tank using one or more fins, optionally wherein the impellor propels the ozone at an angle to the flow of ozone rising from the toilet tank.

6. The ozone mitigation system of any one of claims 1 to 5, wherein the ozone flow restrictor comprises:
a pump configured to create a recirculation path for the ozone within the toilet tank, optionally wherein the recirculation path is substantially vertical.

7. The ozone mitigation system of claim 6, wherein the ozone flow restrictor comprises:
a chamber enclosing a space above a predetermined water level in the toilet tank, wherein the recirculation path passes through the chamber.

8. The ozone mitigation system of any one of the preceding claims, wherein the ozone flow restrictor comprises:
an extended water tube for extending a path of the flow of ozone.

9. The ozone mitigation system of any one of the preceding claims, wherein the ozone flow restrictor comprises:
a fill valve assembly configured to draw ozone from the ozone generator and expel the ozone to the toilet tank at a predetermined position, optionally wherein the fill valve assembly comprises:
a water inlet channel connected to a line supply of water; and
an ozone inlet channel connected to the water inlet via a venturi opening.

10. The ozone mitigation system of claim 9, further comprising:
a filter configured to slow the flow of ozone from the fill valve assembly.

11. The ozone mitigation system of claim 9 or claim 10, wherein the predetermined position is at a bottom of the toilet tank.

12. A method for ozone mitigation in a toilet tank, the method comprising:
providing air to an ozone generator;
providing power to the ozone generator;
generating, at the ozone generator, ozone in a toilet tank in response to the air or the power; and
restricting a flow of the ozone in the toilet tank.

13. The method of claim 12, wherein the ozone generator is submerged in water of the toilet tank.

14. The method of claim 12 or claim 13, further comprising:
providing power to an ozone flow restrictor to restrict the flow of the ozone in the toilet tank.

15. An ozone mitigation system for a toilet, the ozone mitigation system comprising:
an air supply;
a power supply;
an ozone generator configured to generate ozone, in a toilet tank, from the air supply and the power supply; and
an ozone eliminator configured to reduce ozone expelled from the toilet tank.
